# EUROPEAN PATENT SPECIFICATION

(11) **EP 3 991 670 B1**
(45) Date of publication and mention of the grant of the patent: **26.07.2023**
(21) Application number: 21185832.9
(22) Date of filing: 15.07.2021
(51) Int. Cl.: A61B 17/34, A61B 17/00

(54) **HAIR TRANSPLANTER HAVING CORE SHAFT CONVEYING PORTION**
HAARTRANSPLANTATIONSVORRICHTUNG MIT SCHAFTKERNFÖRDERABSCHNITT
DISPOSITIF DE TRANSPLANTATION DE CHEVEUX AYANT UNE PARTIE DE TRANSPORT DE L'ARBRE CENTRAL

(30) Priority: 30.10.2020 KR 20200143497
(43) Date of publication of application: 04.05.2022
(73) Proprietor: OHDAE Corporation, Dalseong-gun, Daegu 42984 (KR)
(72) Inventor: Kim, Byoung Sul, Gyeongsangbuk-do (KR); Lee, Tae Seong, Daegu (KR)
(74) Representative: Grünecker Patent- und Rechtsanwälte PartG mbB

(56) References cited:
- US-A1- 2019 053 827
- US-A1- 2019 223 908

## Description

### BACKGROUND

### 1. Field

The present disclosure relates to a hair transplanter, and more particularly, to a bundle type hair transplanter having a plurality of hair transplantation needles mounted therein in a bundle type.

### 2. Description of the Related Art

A hair transplanter is a medical device that is used for a hair therapy procedure for transplanting hair by harvesting hair follicles from a hair growth area of a scalp and implanting the hair follicles into a bald scalp. A related-art hair transplanter includes a needle (referred to as a "transplantation needle" or a "needle") and a core shaft slidably inserted into the needle, and a bundle type hair transplanter having a plurality of needle channels mounted therein and each including a needle and a core shaft as one unit is used to transplant more hairs.

The related-art hair transplanters may be divided into manual types and automatic types. A manual hair transplanter performs operations of inserting a needle into a scalp by using a force of a user (doctor or operator) or an elastic force of a spring, etc., and retracting the needle, whereas an automatic hair transplanter moves a needle forward by using a force of a motor, inserts the needle into a scalp and implants a hair follicle, and retracts the needle and returns it to an original position by using an elastic force of a spring which supports the needle.

However, in the case of the automatic hair transplanter, when the needle is moved forward by the motor, the needle may move forward at a relatively slow speed and may not pierce a scalp in a single time due to resistance of the scalp. In order to prevent this problem, a user should strongly hold the hair transplanter and there is a problem that much force is required to operate.

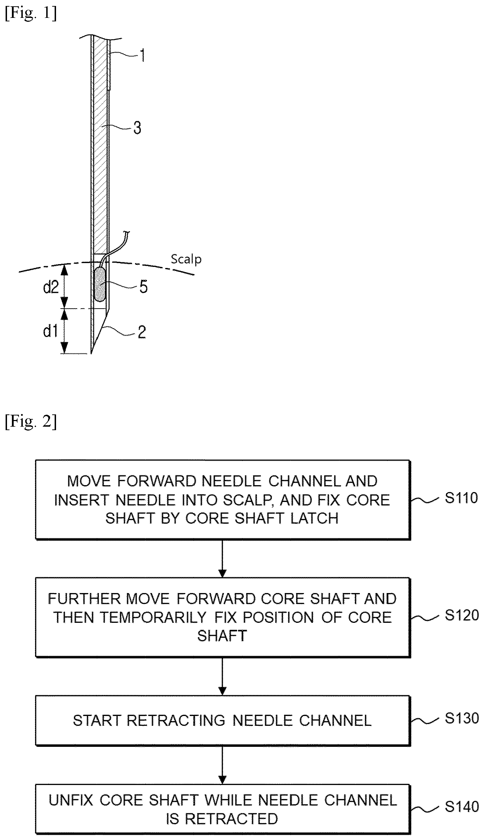

In addition, when a procedure is performed by using the related-art hair transplanter, the needle should be inserted into the scalp by about 7.5 mm-8.5 mm. As shown in FIG. 1, the needle 1 has a penetrating space formed therein, and a core shaft 3 may be inserted into this space. An inclined surface (bevel) 2 is formed on a leading end of the needle 1 to make it easy to insert the needle into the scalp, and the inclined surface 2 has a length d1 of about 3.3 mm. A hair follicle 5 is inserted into the needle 1 to be implanted and has a length of about 4-5 mm.

According to the needle structure described above, if the hair follicle 5 protrudes toward the inclined surface 2, the air follicle 5 may rub against the scalp when the needle is inserted into the scalp, and there is a problem that the hair follicle is destroyed. Therefore, the hair follicle 5 should be positioned inside the inclined surface 2 when the needle is inserted into the scalp. Accordingly, the needle 1 should be inserted into the scalp by at least more than the sum of the length d1 of the inclined surface 2 and the length d2 of the hair follicle 5, and an insertion depth may be about 7.5 mm-8.5 mm. As a result, the needle 1 should be further inserted into the scalp by the length d1 of the inclined surface 2 of the needle in addition to the length d2 of the hair follicle, and a safety problem of a patient such as a damage to the scalp, bleeding, etc. may increase. US2019/0223908 A1 discloses a hair transplanter comprising a needle channel bundle comprising a center shaft and a plurality of needle channels coupled to an outer circumference of the center shaft to be slidable up and down. US 2018/0053827 A1 relates to a hair implanter with an automatically retracting needle.

### SUMMARY

The invention is defined in claim 1. Further embodiments are defined in the dependent claims.

According to an embodiment of the present disclosure, there is provided a hair transplanter which can reduce a penetration depth of a needle inserted into a scalp in comparison with a related-art hair transplanter, and can safely implant a hair follicle into the scalp by further moving forward a core shaft in the needle.

The invention relates to a hair transplantercomprising: a needle channel bundle including a center shaft, and a plurality of needle channels coupled to an outer circumference of the center shaft to be slidable up and down; a first spring configured to push down and move forward one of the plurality of needle channels; and a second spring configured to retract the needle channel, which moves forward, in an upward direction, and each of the needle channels includes a body portion having a tubular inner space, a needle coupled to a lower portion of the boy portion, and a core shaft disposed in the tubular inner space to be slidable. The hair transplanter further comprises a core shaft locking portion which temporarily fixes a position of the core shaft of the needle channel which moves forward by the main spring, wherein, in the state where the core shaft locking portion temporarily fixes the position of the core shaft of the needle channel which moves forward, the hair transplanter is configured to push down the core shaft locking portion by a predetermined distance and to further move forward the core shaft by a predetermined distance.

According to an embodiment of the present disclosure, the hair transplanter can reduce a penetration depth of a needle to a scalp in comparison with a related-art hair transplanter, and can safely implant a hair follicle in the scalp. Accordingly, a damage to a patient's scalp or bleeding can be reduced, and patient's or operator's repulsion against to hair transplantation can be mitigated.

In addition, according to an embodiment of the present disclosure, a hair follicle is implanted in a scalp, and while the needle is retracted by a predetermined distance, the core shaft is temporarily stopped and then the locking of the core shaft is removed. Therefore, the hair follicle can be prevented from being taken out with the needle and can be securely implanted, and a success rate of operation can increase.

### BRIEF DESCRIPTION OF THE DRAWINGS

The above and/or other aspects of the present disclosure will be more apparent by describing certain exemplary embodiments of the present disclosure with reference to the accompanying drawings, in which:
FIG. 1 is a view provided to explain an operation of a needle performed by a related-art hair transplanter;
FIG. 2 is a flowchart provided to explain operations of moving forward and retracting a needle channel according to an embodiment;
FIGS. 3(a), 3(b), 3(c), and 3(d) are views schematically illustrating operations of moving forward and retracting the needle channel according to an embodiment;
FIGS. 4(a), 4(b), and 4(c) are views illustrating an exemplary configuration for moving forward and retracting a core shaft;
FIG. 5 is a perspective view of a hair transplanter according to an embodiment;
FIG. 6 is a cross-sectional view of the hair transplanter according to an embodiment;
FIG. 7 is an exploded perspective view of the hair transplanter according to an embodiment;
FIG. 8 is a view provided to explain a lower cap of the hair transplanter according to an embodiment;
FIG. 9 is a view provided to explain a needle channel bundle according to an embodiment;
FIG. 10(a) is a perspective view of the needle channel according to an embodiment;
FIG. 10(b) is a cross-sectional view of the needle channel according to an embodiment;
FIG. 11 is a cross-sectional perspective view of a center shaft according to an embodiment;
FIG. 12 is a cross-sectional view of the needle channel bundle according to an embodiment;
FIG. 13 is a view provided to explain a configuration for rotating the needle channel according to an embodiment;
FIG. 14 is a view provided to explain a configuration for moving forward the needle channel according to an embodiment;
FIGS. 15(a) and 15(b) are views provided to explain operations of locking and unlocking of a push block according to an embodiment;
FIG. 16 is a view provided to explain a core shaft locking portion according to an embodiment;
FIGS. 17(a) and 17(b) are views provided to explain an operation of further moving forward of the core shaft locking portion according to an embodiment;
FIG. 18 is a view provided to explain operations of locking and unlocking of the needle channel and the core shaft locking portion according to an embodiment; and
FIGS. 19(a), 19(b), 19(c), 19(d), and 19(e) are views provided to explain operations of moving forward and retracting the needle channel according to an embodiment.

### DETAILED DESCRIPTION OF EMBODIMENTS

Exemplary embodiments will now be described more fully with reference to the accompanying drawings to clarify aspects, other aspects, features and advantages of the present disclosure. The exemplary embodiments may, however, be embodied in many different forms and should not be construed as limited to the exemplary embodiments set forth herein. Rather, the exemplary embodiments are provided so that this disclosure will be thorough and complete, and will fully convey the scope of the application to those of ordinary skill in the art.

It will be understood that when an element is referred to as being "on" (or "under", "on the right of', or "on the left of') another element, the element can be directly on (or "under", "on the right of', or "on the left of') another element or a third element therebetween.

The expressions such as "upper", "lower", "left", "right", "front", "rear", etc. used in the specification to explain a position relationship between elements do not mean a directions or location as an absolute criterion, and are relative expressions used for convenience of explanation with reference to a corresponding drawing when the present disclosure is explained with reference to each drawing.

In the detailed description, when a certain element is referred to as being connected (or coupled, secured, attached) to another element, the element may be directly connected (or coupled, secured, attached) to another element or may be indirectly connected (or coupled, secured, attached) to another element with a third element interposed therebetween. In the drawings, lengths, thicknesses, or wideness of elements are exaggerated for easy understanding of technical features, and a relative size of a certain element to another element may vary according to a specific embodiment.

As used herein, the singular forms are intended to include the plural forms as well, unless the context clearly indicates otherwise. It will be further understood that the terms "comprise" and/or "comprising," when used in this specification, do not preclude the presence or addition of one or more other components.

If the terms such as 'first' and 'second' are used to describe elements, these elements should not be limited by such terms. These terms are used for the purpose of distinguishing one element from another element only. The exemplary embodiments include their complementary embodiments.

In the detailed description, a "forward movement" of a certain element means that the element moves toward a patient's scalp, and a "backward movement" or "retracting" means that the element moves far away from the patient's scalp. In the detailed description, a "downward direction" of the hair transplanter refers to a longitudinal direction of the hair transplanter in which a needle channel moves forward, and an "upward direction" refers to a longitudinal direction of the hair transplanter in which the needle channel is retracted. In the detailed description, a "forward direction" of the hair transplanter refers to a direction in which the needle channel moves forward, and a "backward direction" refers to a direction in which the needle channel is retracted. In addition, in the detailed description, a "radially" inward or outward direction refers to a radially inward or outward direction with reference to a center axis of the hair transplanter in the longitudinal direction of the hair transplanter.

Hereinafter, exemplary embodiments will be described in greater detail with reference to the accompanying drawings. The matters defined in the description are provided to assist in a comprehensive understanding of the exemplary embodiments. However, it is apparent that the exemplary embodiments can be carried out by those of ordinary skill in the art without those specifically defined matters. In the description of the exemplary embodiment, certain detailed explanations of related art are omitted when it is deemed that they may unnecessarily obscure the essence of the inventive concept.

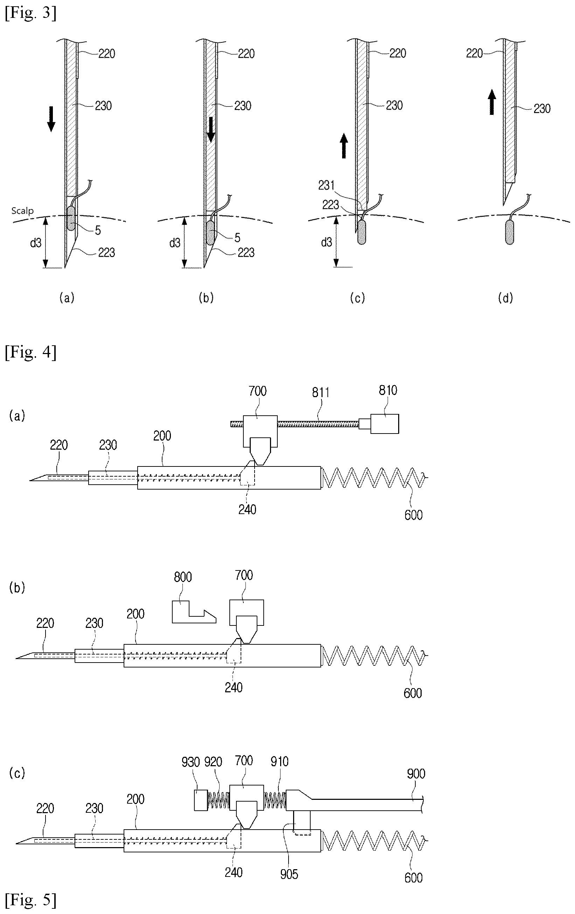

FIG. 2 is a flowchart provided to explain operations of moving forward and retracting a needle channel according to an embodiment of the present disclosure, and FIGS. 3(a), 3(b), 3(c), and 3(d) are views schematically illustrating the operations of moving forward and retracting the needle channel at each step of FIG. 2.

In an embodiment of the present disclosure, the "needle channel" includes one needle 220 and a core shaft 230, and the hair transplanter of the present disclosure may be a bundle type hair transplanter having a plurality of needle channels mounted therein. The operations of moving forward and retracting one needle channel will be described with reference to FIG. 2 and FIGS. 3(a), 3(b), 3(c), and 3(d) for convenience of explanation.

Referring to FIG. 2 and FIGS. 3(a), 3(b), 3(c), and 3(d), at step S 110 of FIG. 2, the needle channel is moved forward by a predetermined distance (for example, a "first predetermined distance") and the needle is inserted into a patient's scalp. That is, as shown in FIG. 3(a), the needle channel is moved forward and a leading end of the needle 220 is inserted into the patient's scalp. In an embodiment, when a height of an inclined surface 223 of the leading end of the needle 220 is about 3 mm-3.5 mm, a penetration depth d3 of the needle 220 may be about 5 mm-5.5 mm, and a portion of a hair follicle 5 received in the needle 220 is putted into the scalp. At step S 110, the needle channel may be moved forward by the first predetermined distance, and then, the core shaft 230 may be restrained from being freely moved by being locked into a core shaft locking portion (not shown).

The operation of moving the needle channel forward by the first predetermined distance at step S110 may be manually controlled by a user's (or doctor's or operator's) force, but preferably, the needle may be made to be inserted into the scalp for a short moment in a single time by using a spring having a great elastic force. In an embodiment, the penetration depth d3 of the needle 220 may be about 5 mm to 5.5 mm, but according to a specific embodiment, the penetration depth d3 may vary.

Next, at step S120, the core shaft 230 may be further moved forward by a predetermined distance (for example, a "second distance"), and then, a position of the core shaft 230 is temporarily fixed. For example, the core shaft 230 may be moved forward and then may be fixed by moving forward the core shaft fixing portion which fixes the core shaft 230 and temporarily fixing the core shaft fixing portion. As shown in FIG. 3(b), the hair follicle 5 may be completely pushed into the scalp by further moving forward the core shaft 230 by the second distance at step S120. The core shaft 230 may further be moved forward to the extent that the hair follicle 5 is completely inserted into the scalp, and for example, the additional forward movement of the core shaft 230 may be 3 mm to 3.5 mm, but may vary according to a specific embodiment.

Thereafter, at step S130, a backward movement of the needle channel is started. The backward movement of the needle channel may be manually performed by a user's (or doctor's or operator's) force, or may be automatically or semiautomatically performed by a driving motor or a spring. At step S130, the needle 220 is retracted but the core shaft 230 is locked into the core shaft locking portion and is temporarily stopped as shown in FIG. 3(c) and accordingly, the hair follicle 5 is not taken out along with the needle 220 while the needle 220 is taken out of the scalp, and is safely implanted in the scalp.

Thereafter, at step S 140, when the needle channel is retracted by a predetermined distance ("third predetermined distance"), the core shaft 230 is unlocked from the core shaft locking portion and is retracted in the upward direction along with the needle 220. In this case, the third predetermined distance may be a distance by which the needle 220 is retracted until a lower end 231 of the core shaft 230 starts being exposed to the inclined surface 223 of the needle 220, as shown in FIGS. 3(c) and 3(d), but the third predetermined distance may be differently set according to a specific embodiment. As described above, the needle 220 and the core shaft 230 are retracted in the upward direction all together, such that the needle channel is returned to its initial position (that is, a standby position before the needle channel is moved forward).

According to the hair transplanter of the present disclosure described above, the needle channel is moved forward to the extent that the hair follicle 5 in the needle 220 penetrates through the scalp only in part (FIG. 3(a)), and then, the hair follicle 5 is completely implanted in the scalp by an additional forward movement of the core shaft 230 (FIG. 3(b)). Compared to the related-art technology of FIG. 1, the hair transplanter of the present disclosure can reduce a penetration depth of the needle 220 to the scalp and can reduce a damage to the patient's scalp. In addition, according to the present disclosure, the hair follicle 5 is implanted in the scalp, and, while the needle 220 is retracted by a predetermined distance (third predetermined distance), the core shaft 230 is temporarily stopped and then is unlocked, so that the hair follicle 5 can be prevented from being taken out along with the needle 220 and the hair follicle 5 can be surely implanted, and a success rate of a procedure can increase.

In order to perform the additional forward movement of the core shaft 230 right after the forward movement of the needle 220 and to perform the temporary stopping operation of the core shaft 230 when the needle 220 is retracted, the core shaft locking portion which locks the core shaft 230 may further be moved forward and may be temporarily stopped.

FIGS. 4(a), 4(b), and 4(c) are views schematically illustrating various embodiments of the present disclosure in which the core shaft 230 is further moved forward and is temporarily stopped by further moving forward the core shaft locking portion 700 and temporarily stopping the core shaft locking portion 700.

In an embodiment of FIG. 4(a), the needle channel 200 includes the needle 220 protruding from a leading end thereof, and the core shaft 230 slidably disposed in the needle 220. A lever 240 may be coupled to one end of the core shaft 230, and accordingly, the core shaft 230 and the lever 240 may integrally slide forward and backward.

In the illustrated embodiment, the needle channel 200 may be moved forward by a main spring 600, and may be retracted by a spring or a conveyance bar (not shown). The spring may be disposed between a front end of the needle channel 200 and a lower case of the hair transplanter, and may be configured to always press the needle channel 200 in a direction of retracting the needle channel 200.

In this case, the lever 240 may be configured to be locked by the core shaft locking potion 700, and may temporarily fix the core shaft 240 not to move when the needle channel 200 is retracted. The core shaft locking portion 700 may be connected to a rotary shaft 811 of a driving motor 810 to be slidable, and the core shaft 230 may further be moved forward by the second distance by further moving forward the core shaft locking portion 700 by the second predetermined distance by using the driving motor 810. In addition, while the needle channel 200 is retracted, the driving motor 810 may temporarily stop its operation and may temporarily stop the core shaft locking portion 700, and then, the main spring 600 may be retracted, and accordingly, the needle channel 200 may be retracted by the third predetermined distance. Then, the core shaft 230 may be retracted by moving the core shaft locking portion 700 in the backward direction by driving the driving motor 810.

FIG. 4(b) illustrates another exemplary configuration for enabling an additional forward movement and a temporary stop operation of the core shaft 230. In this embodiment, the needle channel 200 may be moved forward by the main spring 600 and may be retracted by a spring (not shown). The lever 240 may be configured to be locked by the core shaft locking portion 700 and to restrict a movement. The core shaft locking portion 700 may further be moved forward by the second predetermined distance by a forward movement of a push bar (not shown), and may be fixed in the further moved state.

When the main spring 600 is retracted, the needle channel 200 may also be retracted, but the core shaft locking portion 700 may be temporarily fixed, and then, after the needle channel 200 is retracted by the third predetermined distance, the core shaft locking portion 700 may be unfixed and the core shaft 230 may be unlocked and may be retracted.

FIG. 4(c) illustrates still another embodiment in which the needle channel 200 may be moved forward by the main spring 600 and may be retracted by a conveyance bar 900. A first auxiliary spring 910 may be disposed between the core shaft locking portion 700 and the conveyance bar 900, and a second auxiliary spring 920 may be disposed at a front of the core shaft locking portion 700. The second auxiliary spring 920 may be fixed to a certain member 930 like a frame or a case of the hair transplanter and may be supported thereon. The first auxiliary spring 910 may be configured to have a greater elastic force than that of the second auxiliary spring 920.

In this embodiment, the core shaft locking portion 700 may further be moved forward by the second predetermined distance by a forward movement of the conveyance bar 900. In this case, since the elastic force of the first auxiliary spring 910 is greater than that of the second auxiliary spring 920, the core shaft locking portion 700 may further be moved forward. When the needle channel 200 is retracted, the conveyance bar 900 may also be retracted while the needle channel 200 is retracted by the third predetermined distance, but the first auxiliary spring 910 still presses the core shaft locking portion 700. Therefore, the core shaft locking portion 700 is not retracted in the backward direction and remains still, and, when the conveyance bar 900 is further retracted and the elastic force of the second auxiliary spring 920 becomes greater than that of the first auxiliary spring 910, the core shaft locking portion 700 is pushed backward by the second auxiliary spring 920 and the core shaft 230 is retracted.

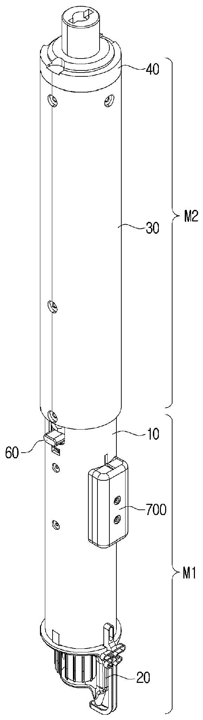

As described above, there are various embodiments in which the core shaft 230 is further moved forward right after the needle 220 is moved forward and the core shaft 230 is retracted with a delay when the needle 200 is retracted, and hereinafter, a specific and exemplary device configuration of the hair transplanter according to the embodiment of FIG. 4(b) will be described with reference to FIGS. 5, 6, 7, 8, 9, 10(a), 10(b), 11, 12, 13, 14, 15(a), 15(b), 16, 17(b), 17(b), 18, 19(a), 19(b), 19(c), 19(e), and 19(e).

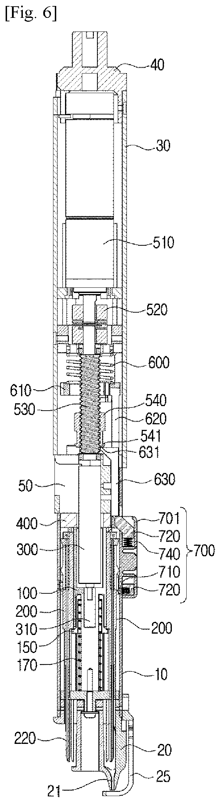

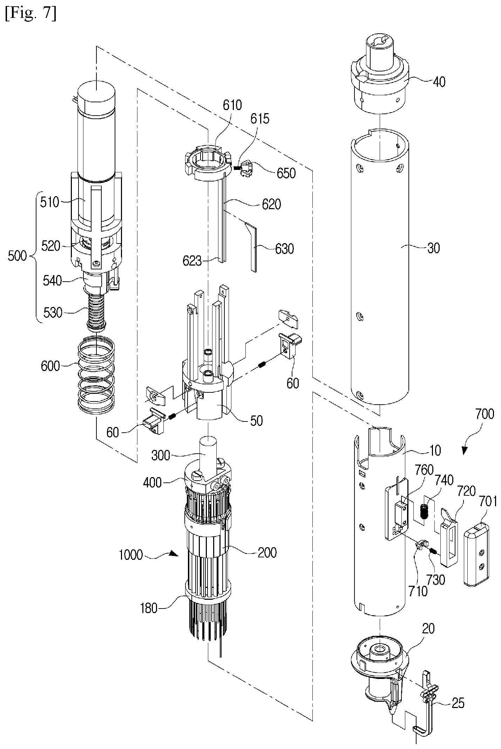

FIG. 5 is a perspective view of the hair transplanter according to an embodiment of the disclosure, FIG. 6 is a cross-sectional view of the hair transplanter, and FIG. 7 is an exploded perspective view of the hair transplanter. Referring to FIGS. 5, 6, and 7, the hair transplanter according to an embodiment may have an exterior of a long cylindrical shape, such that it can be used by being held with user's (for example, doctor's or operator's) hand.

The hair transplanter may include a lower module M1 and an upper module M2. The lower module M1 may be cased with a lower case 10 and a lower cap 20 coupled to a lower portion of the lower case 10, and the upper module M2 may be cased with an upper case 30, an upper cap 40 coupled to an upper portion of the upper case 30, and a bracket 50 coupled to a lower portion of the upper case 30.

It is preferable that the lower module M1 and the upper module M2 are separably coupled to each other to make it easy to mount and dismount a needle channel bundle 1000 disposed in the lower module M1. In the illustrated embodiment, a module coupling portion 60 in a lever type may be installed on the bracket 50 of the upper module M2, and the modules M1, M2 may be separated from each other by a user pushing the lever.

The needle channel bundle 1000 in which a plurality of needle channels 200 for transplanting hair follicles are coupled in a bundle type may be rotatably disposed in the lower case 10 of the lower module M1. The needle channel bundle 1000 may include a cylindrical center shaft 100 and a plurality of needle channels 200 radially arranged on an outer circumference of the center shaft 100 at equal intervals.

A first driving motor 300 may be disposed on an upper portion of the needle channel bundle 1000. A driving shaft of the first driving motor 300 may be coupled to a center of the center shaft 100 by a coupling 310. Accordingly, the needle channel bundle 100 may be rotated by driving of the first driving motor 300. The first driving motor 300 may be implemented by a step motor, etc., for example, and may rotate the needle channel bundle 1000 by a predetermined angle. In an embodiment, the needle channel bundle 1000 may be rotated by the first driving motor 300 by an angle corresponding to an interval of one needle channel 200 at a time. For example, in the illustrated embodiment, when 18 needle channels 200 are attached to the outer circumference of the center shaft 100, the first driving motor 300 is configured to rotate the needle channel bundle 100 by 20 degrees by one driving.

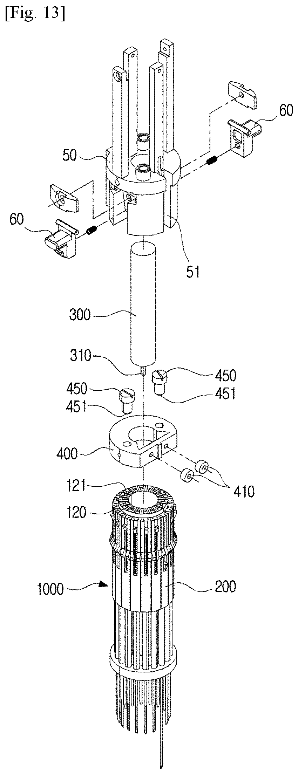

In an embodiment, a ball plunger (451 in FIG. 13) may be disposed in contact with an upper surface of the needle channel bundle 100 so as to rotate the needle channel bundle 100 by a predetermined angle at a time. The ball plunger 451 may be coupled to and supported on a ball plunger support portion 400 disposed on an upper portion of the needle channel bundle 1000.

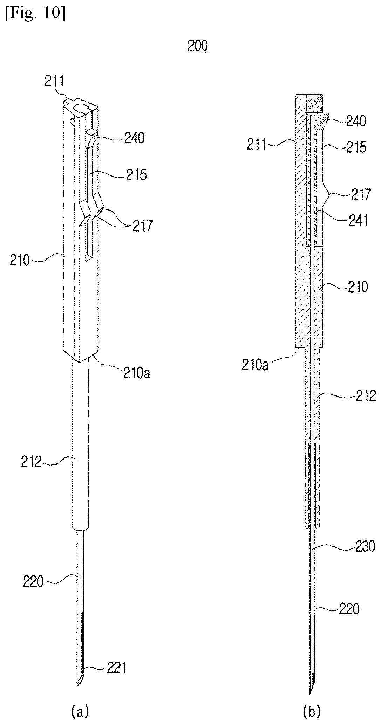

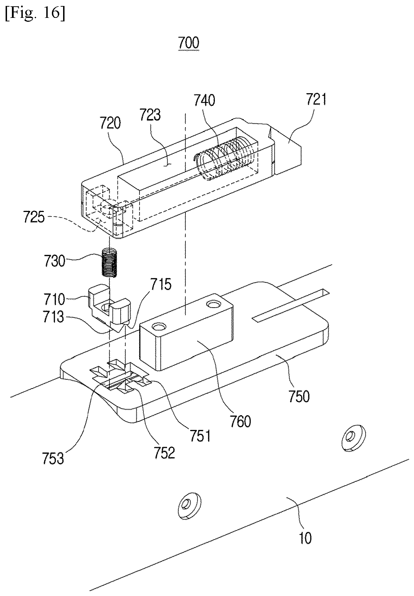

The core shaft locking portion 700 is disposed on a side surface of the lower case 10. The core shaft locking portion 700 performs the role of locking a core shaft (230 of FIG. 10) slidably disposed in the needle channel 200 to temporarily restrain the core shaft from being moved. A specific configuration of the core shaft locking portion 700 will be described below with reference to FIGS. 16, 17(b), 17(b), and 18.

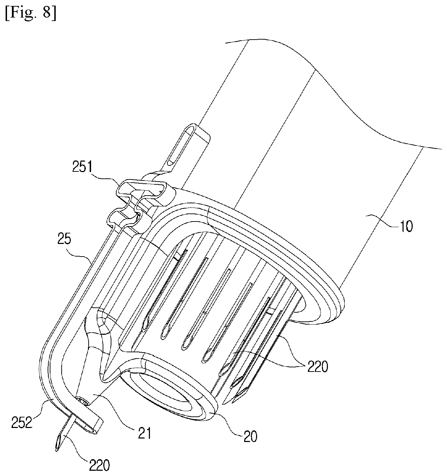

The lower cap 20 is removably coupled to a lower end of the lower case 10 to cover the lower end of the lower case 10. In this regard, FIG. 8 illustrates the lower cap 20 of the hair transplanter according to an embodiment. In the embodiment of FIG. 8, the lower cap 20 is configured in a frame structure to allow a user to view the needle 220 of each needle channel 200. A nozzle 21 may be formed on a lower surface of the lower cap 20 to allow the needle 220 of one needle channel 200 to pass therethrough, and one needle 220 may protrude downward by a predetermined length through the nozzle 21.

In an embodiment, the lower cap 20 includes a guide member 25. The guide member 25 is a member that has a slot 252 and has a long and curved shape, and has one end fixed to a certain position of the lower cap 20 and the other end covering a leading end of the nozzle 21. The needle 220 protruding through the nozzle 21 protrudes through the slot 252 of the guide member 25 and there is no interference between the needle 220 and the guide member 25.

In an embodiment, at least a portion of the guide member 25 may be formed as an elastic structure 251 to absorb or reduce an impact. The elastic structure 251 may be formed by bending at least a portion of the guide member 25 in an S shape or a zigzag shape, as shown in FIG. 8, but this should not be considered as limiting. The elastic structure 251 may be implemented by a certain shape or a certain material that can mitigate or absorb an impact.

When the guide member 25 is installed on the lower cap 20, the user can insert the needle 220 into a patient's scalp with the guide member 25 being in close contact with a treatment position of the patient's scalp. Therefore, there are advantages that the needle can be more stably inserted and an impact exerted to the patient when the needle is inserted can be mitigated.

Referring back to FIGS. 5, 6, and 7, the upper module M2 may be cased with the upper case 30, and the upper cap 40 and the bracket 50 which are attached to the upper end and the lower end of the upper case 30, respectively. The upper module M2 may include a main spring 600, a push block 610, and a main push bar 620 to push down one needle channel to move the same forward, and may further include a sliding block 540 to pull up the needle channel, which has moved down, and to return the needle channel to its initial state (that is, a state before the needle channel is pushed by the main spring).

An upper end of the main spring 600 may be attached to a certain position of the upper case 30 and fixed thereto. The push block 61 which has a ring shape is attached to a lower end of the main spring 600, and the main push bar 620 is attached to the push block 610 in a longitudinal direction of the hair transplanter. The main spring 600 may be retracted and pressed by a certain member, and, when the pressing is released, the main spring 600 may be moved forward by an elastic force, and accordingly, the main push bar 620 may push the needle channel 200 and may move forward the needle channel 200. The main spring 600 may have a strong elastic force, and accordingly, the needle channel 200 may be pushed by the main push bar 620 and may be moved forward instantaneously for a short moment.

In an embodiment, a driving portion 500 may include a second driving motor 510, a coupling 520, a lead screw 530, and the sliding block 540. The second driving motor 510 may be implemented by a certain motor having a driving shaft rotating in both directions, and a driving shaft of the second driving motor 510 may be secured to the lead screw 530 by the coupling 520. The lead screw 530 may have a screw thread formed on an outer circumference thereof, and the sliding block 540 may be coupled to the lead screw 530 to move up and down by rotation of the lead screw 530. In this case, since the main spring 600 has a diameter larger than those of the lead screw 530 and the sliding block 540, a movement of the main spring 600 may not interfere with a movement of the lead screw 530 or the sliding block 540.

The main push bar 620 may have a slot formed in a longitudinal direction, and an auxiliary push bar 630 is mounted in the slot to be freely slidable in the longitudinal direction. A protruding piece 631 is formed on an upper end of the auxiliary push bar 630 to protrude in the radially inward direction, and the protruding piece 631 is configured to be in contact with a lower end 541 of the sliding block 540. A lower end of the auxiliary push bar 630 is configured to be in contact with the core shaft locking portion 700. According to this configuration, when the sliding block 540 moves down and the lower end 541 of the sliding block pushes down the auxiliary push bar 630, the auxiliary push bar 630 pushes the core shaft locking portion 700, thereby further moving forward the core shaft locking portion by a predetermined distance.

A power line and a control line for applying power to the driving portion 500 and controlling an operation may be connected to the upper module M2 from the outside, and a wire connector (not shown) for this may be formed in the upper module M2. In addition, although not shown, a power line and/or a control line for driving rotation of the needle channel bundle 1000 may be connected to the lower module M1 from the outside, and a wire connector for this (not shown) may be formed in the lower module M1.

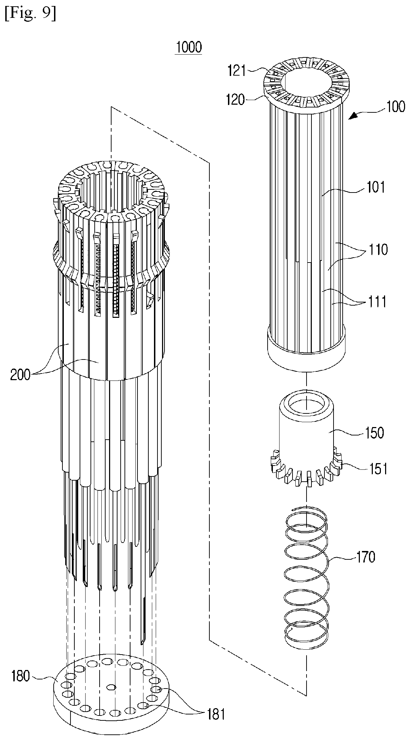

FIG. 9 is a view to explain the needle channel bundle 1000 according to an embodiment. Referring to FIG. 9, the needle channel bundle 1000 may include the center shaft 100, the plurality of needle channels 200 coupled to the outer circumference of the center shaft 100 to be slidable up and down, an elevation portion 150 mounted in the center shaft 100 to be slidable, and a spring 170 for elastically supporting the elevation portion.

FIG. 10(a) is a perspective view of the needle channel 200 according to an embodiment, and FIG. 10(b) is a cross-sectional view of the needle channel 200.

Referring to the drawings, the needle channel 200 may include a body potion 210 having a tubular inner space, the needle 220 coupled to a lower portion of the body portion 210, and the core shaft 230 disposed in the tubular inner space to be slidable.

The body portion 210 includes a vertical protrusion 211 formed along one side surface in a vertically longitudinal direction. The vertical protrusion 211 may protrude toward the center shaft 100, that is, in the radially inward direction, and the vertical protrusion 211 is configured to have a protruding shape to be fitted into a groove of a slide rail 111 of the center shaft 100. Accordingly, the vertical protrusion 211 of the body portion 210 is press-fitted into the slide rail 111 of the center shaft 100, such that the needle channel 200 can slide up and down along the slide rail 111.

The body portion 210 includes a small diameter portion 212 of a diameter smaller than the diameter of the body portion 210. The body portion 210 and the small diameter portion 212 may be integrally formed with each other, and a step portion 210a is formed between the body portion 210 and the small diameter portion 212.

The needle 220 may be inserted and coupled into a lower portion of the body portion 210, and the core shaft 230 is slidably disposed in the inner space of the needle 220. The needle 220 may have a slot 221 formed on a side surface of a lower end thereof and opened outward to easily thread the needle with a hair follicle. The core shaft 230 is inserted and disposed in the inner space of the needle 220, but is disposed such that the lower end of the core shaft 230 is lower than the uppermost end of the slot 221, but does not protrude below the needle 220. The slot 221 is formed in the radially outward direction of the needle channel bundle 100, and provides convenience when an operator transplants a hair follicle.

The body portion 210 comprises a slot 215. The slot 215 is an opening formed in a longitudinally and radially-outward direction in the body portion 210. A lever 240 may be disposed in the slot 215 to be slidable up and down along the slot 215, and the lever 240 may have one side surface protruding more outwardly than the slot 215 (that is, in the radially outward direction). An upper end of the core shaft 230 may be coupled to the lever 240, and accordingly, the lever 240 and the core shaft 230 may integrally slide up and down.

A spring 241 is fitted over the circumference of the core shaft 230 within the slot 215. An upper end of the spring 241 may be supported by a lower surface of the lever 240, and a lower end of the spring 241 may be supported by a bottom surface of the slot 215. Accordingly, when a force is applied to the lever 240 and the lever 240 and the core shaft 230 move down, and then, the force applied to the lever 240 is removed, the core shaft 230 may move up and may return to its original position due to an elastic force of the spring 241.

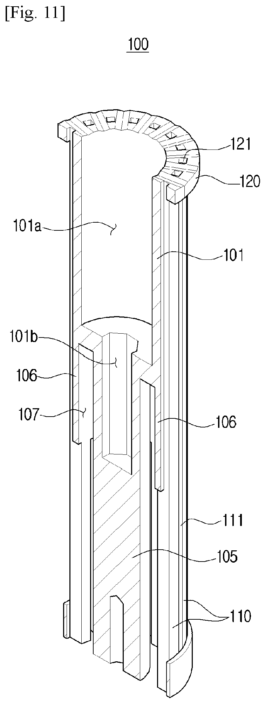

FIG. 11 is a cross-sectional view of the center shaft 100 according to an embodiment. The center shaft 100 may have a cylindrical shape. The center shaft 100 includes an outer wall 110 surrounding an outer circumference thereof, and a plurality of slide rails 111 are formed on the outer wall 110 radially at equal intervals with reference to the center of the center shaft. In an embodiment, the slide rail 111 may be engraved in the vertically longitudinal direction.

A flange 120 having a diameter larger than that of the center shaft 100 is formed on an upper end of the center shaft 100. The flange 120 may be integrally formed with the center shaft 100, or the flange 120 and the center shaft 100 may be separately manufactured and then the flange 120 may be attached to the upper end of the center shaft 100. As described above, since the flange 120 having the larger diameter is positioned on the upper end of the center shaft 100, the needle channels 200 may not move up any more, and accordingly, the flange 120 may serve as a stopper to restrict an upward sliding movement range of the needle channel 200.

The center shaft 100 may include an inner large diameter portion 101 formed inside the outer wall 110, and an inner small diameter portion 105 integrally extended downward from the inner large diameter portion. 101 The inner large diameter portion 101 may be integrally formed with the inner surface of the outer wall 110. The inner large diameter portion 101 includes a motor receiving groove 101a having an upper portion opened, and a coupling receiving groove 101b additionally formed under the motor receiving groove 101a. The motor receiving groove 101a may receive at least a portion of the first driving motor 300, and the coupling receiving groove 101b may receive the coupling 310 of the first driving motor 300, and the coupling 310 and the coupling receiving groove 101b are secured to each other, such that the center shaft 100 is rotated by the first driving motor 300.

The inner small diameter portion 150 may be extended downward from the inner large diameter portion 101. An upper portion of the inner small diameter portion 105 may be surrounded by a skirt portion 106 having the same outer diameter as that of the inner large diameter portion 101, and accordingly, a separation space 107 of a predetermined gap is formed between the inner small diameter potion 105 and the skirt portion 106.

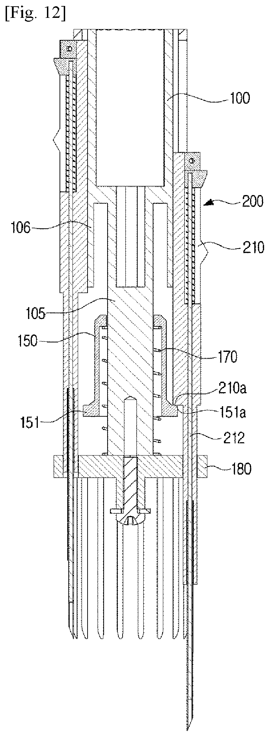

FIG. 12 is a cross-sectional view of the needle channel bundle, schematically illustrating a coupling relationship between the center shaft 100 and the needle channel 200 according to an embodiment. Referring to FIGS. 9, 10(a), 10(b), 11, and 12, the vertical protrusion 211 of each needle channel 200 is fitted into the sliding rail 111, such that the needle channel 200 is slidably mounted on the center shaft 100, and in this state, a guide ring 180 is mounted around a lower portion of the center shaft 100. The guide ring 180 includes a penetrating hole 181 through which the small diameter potion 212 of each needle channel 200 penetrates, and may serve to guide stable mounting and a sliding movement of each needle channel 200.

The elevation portion 150 is a member of a cylindrical shape, and is fitted over the inner small diameter portion 105 of the center shaft 100 to be slidable. The elevation portion 150 includes a projection 151 protruding in the radially outward direction. The number of projections 151 is the same as the number of needle channels 200 mounted on the center shaft 100, and each projection 151 may protrude to the extent that the projection 151 comes into contact with the step portion 210a of the needle channel 200 and interferes therewith.

The spring 170 is disposed between the elevation portion 150 and the guide ring 180. An upper end of the spring 170 supports the elevation portion 150, and a lower end is supported on an upper surface of the guide ring 180, and accordingly, the spring 170 always applies a force to elevate the elevation portion 150.

In this configuration, when one needle channel 200 is moved down by the main push bar 620, the step portion 210a of the needle channel 200 pushes the projection 151 of the elevation portion 150 and the elevation portion 150 also moves down. When the main push bar 620 ascends, the force pushing down the needle channel 200 is removed, and thus, the spring 170 pushes up the elevation portion 150 and the needle channel 200 due to the elastic force of the spring 170, and the needle channel 200 moves up and returns to its original position.

In an alternative embodiment, every needle channel 200 may include a spring (not shown), instead of the elevation portion 150 and the spring 170. For example, every needle channel 200 may include a spring surrounding the small diameter portion 212, and an upper end of the spring may be supported by the step portion 210a and a lower end may be supported by the guide ring 180. Accordingly, when a certain needle channel 200 moves down and then an external force is removed, the needle channel may move up again and return to its original position due to the elastic force of the spring.

FIG. 13 is an exploded perspective view illustrating some elements of the hair transplanter according to an embodiment.

Referring to FIG. 13, the bracket 50 has a substantially cylindrical shape having an inner space formed therein, and an upper portion of the bracket 50 may be coupled and fixed to the lower end of the upper case 30 to form a portion of the upper module M2. A guide groove 51 is formed on one side surface of the bracket 50 to guide the main push bar 620.

Module coupling portions 60 may be installed on both side surfaces of the bracket 50 to be coupled with the lower module M1. A well-known certain securing method may be used to couple the lower module M1 and the upper module M2.

The ball plunger support portion 400 may be coupled to a lower portion of the bracket 50. The ball plunger support portion 400 may have a ring shape having a predetermined thickness, and may be disposed to have the first driving motor 300 to penetrate through a center penetrating area thereof. One side surface of the ball plunger support portion 400 may be cut not to interfere with a movement of the main push bar 620. A guide roller 410 may further be included to guide a longitudinal movement of the main push bar 620.

The ball plunger support portion 400 includes at least one ball plunger 450 including a ball 451 protruding downward. The ball plunger 450 may include the ball 451 having a portion exposed in the downward direction, and a spring (not shown) disposed in the ball plunger 450 to elastically support the ball. Although two ball plungers 450 are illustrated in the drawing, the number of ball plungers 450 or installation positions thereof are not limited.

In an embodiment, as shown in FIGS. 9, 10(a), 10(b), and 11, an upper surface of the center shaft 100, that is, an upper surface of the flange 120, may have a concavo-convex shape. That is, a groove portion and a protrusion portion are alternately formed on the upper surface of the flange 120, and the groove portion 121 is formed on every position of the slid rail 111 in the vertically upward direction. The ball plunger support portion 400 may be disposed such that the ball 451 of the ball plunger 450 comes into contact with the concavo-convex portion of the flange 120 and the ball 451 elastically presses the concavo-convex surface. Since the ball 451 is operated in the direction of being seated in the groove portion of the flange 120, it is guaranteed that the needle channel bundle 1000 is regularly rotated by a predetermined angle at a time.

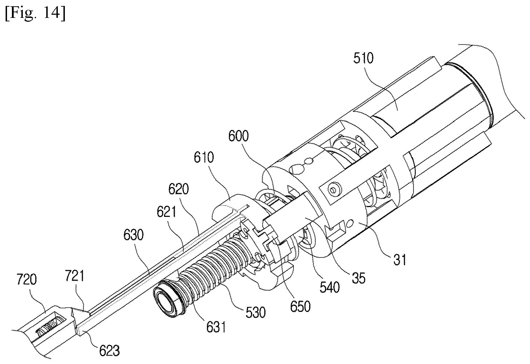

FIG. 14 is a view to explain a configuration of moving forward the needle channel according to an embodiment. Referring to the drawing, the push block 610 of a ring shape is coupled to a lower end of the main spring 600, and the main push bar 620 is mounted on the push block 610 in the longitudinal direction. The main push bar 620 has a bar shape of a predetermined length and has a slot 621 formed thereon in the longitudinal direction, and the auxiliary push bar 630 is mounted in the slot 621. The auxiliary push bar 630 is freely slidable within the slot 621, and accordingly, the auxiliary push bar may move independently regardless of a movement of the main push bar 620.

According to this configuration, when the main spring 600 moves down by the elastic force, the push block 610 and the main push bar 620 which are integrally couped with the main spring also move down, and a leading end 623 of the main push bar 620 pushes an upper portion of the needle channel 200 to move down the corresponding needle channel 200.

The sliding block 540 may move down by rotation of the lead screw 530, and, when the sliding block 540 moves down and the lower end 541 of the sliding block pushes the protruding piece 631 of the auxiliary push bar 630, the auxiliary push bar 630 may move down and may push the core shaft locking portion 700 to further move the core shaft locking portion by a predetermined distance.

In this case, the sliding block 540 is mounted on the lead screw 530 which is rotated by the second driving motor 510. Since the diameter of the main spring 600 is larger than the sliding block 540 and the push block 610 has a ring shape, interference does not occur between a movement of the main spring 600 or the push block 610 and a movement of the sliding block 540.

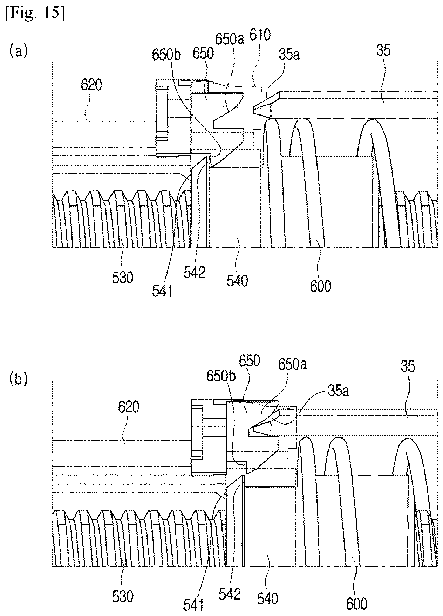

In an embodiment, the push block 610 may further include a locking portion 650. As shown in FIG. 7, a spring 615 may be interposed between the locking portion 650 and the push block 610, and the push block 610 may move within a predetermined length in the radial direction. The sliding block 540 includes a step portion (542 of FIG. 15) formed on one side surface thereof, and the step portion 542 may restrict a movement of the push block 610 by locking the locking portion 650. In addition, the hair transplanter according to the present disclosure may include an unlocking rod 35 of a predetermined length formed in the radially outward direction of the main spring 600. The unlocking rod 35 may have one end coupled and fixed to a certain member of the upper module M2 of the hair transplanter, and the other end configured to be in contact with the locking portion 650.

FIG. 15(a) is a view to explain operations of locking and unlocking of the push block according to an embodiment, and FIG. 15(b) is a view to explain operations of locking and unlocking of the push block according to an embodiment.

FIG. 15(a) illustrates a state in which the sliding block 540 locks the push block 510. In an embodiment, the locking portion 650 includes a locking projection 650b formed in the radially inward direction, and the projection 650b is locked into the step portion 542 of the sliding block 540. Accordingly, when the sliding block 540 is retracted (that is, moves to the right in FIG. 15(a)), the push block 610 is retracted and the main spring 600 is compressed, becoming shorter.

Thereafter, when the sliding block 540 is further retracted as shown in FIG. 15(b), an inclined surface 650a formed on a radial outside of the locking portion 650 comes into contact with an inclined surface 35a of a leading end of the unlocking rod 35, and the locking portion 650 moves along the inclined surface 35a of the rod 35 in the radially outward direction (that is, the upward direction in FIG. 15(b)), such that locking between the step portion 542 of the sliding block 540 and the projection 650b is removed. Accordingly, soon after unlocking, the push block 610 immediately moves forward (that is, to the left in FIG. 15(b)) due to the strong elastic force of the main spring 600.

Hereinafter, the core shaft locking portion 700 and an operation thereof will be described with reference to FIGS. 16, 17(b), 17(b), and 18.

FIG. 16 is a view to explain the core shaft locking portion according to an embodiment. Referring to FIG. 16, the core shaft locking portion 700 may be installed on one side surface of the lower case 10 as shown in FIGS. 5, 6, and 7, and may perform the function of further moving forward the core shaft 230 in the downward direction after the needle channel 200 is pushed downward by the main spring 600, and also, may perform the function of temporarily stopping the movement of the core shaft 230 within the needle channel 200 while the needle channel 200 is moving up.

In an embodiment, the core shaft locking portion 700 includes a case 701, and a latch 710 and a lock main body 720 which are disposed in the case. The lock main body 720 may have a leading end 721 integrally formed with an upper portion thereof in the longitudinal direction, and having a narrow width, and a slot 723 is formed on the center of the main body 720. In addition, the lock main body 720 has a latch receiving space 725 to allow the latch 710 to be inserted thereinto, and an elastic member 730 such as a spring is interposed in the space. Accordingly, the latch 710 is disposed within such a distance that the latch 710 interferes with a vertical movement of the lever 240, and may be elastically retracted in the radially outward direction of the hair transplanter.

A support portion 750 having a flat surface is formed on the lower case 10 of the hair transplanter to support the lock main body 720, and a protruding guide 760 is formed on a center of the support portion 750. The protruding guide 760 may be inserted into the slot 723 of the lock main body 720, and accordingly, may restrict a sliding movement of the lock main body 720 in the longitudinal direction, and simultaneously, may guide the movement. In addition, an elastic member such as a spring 740 may be interposed between the slot 723 and the protruding guide 760, and accordingly, the lock main body 720 has elasticity to move in a retracting direction when there is no external force.

In an embodiment, a penetrating hole 753 is formed on a surface of the support portion 750 to allow the latch 710 to penetrate therethrough, and a first seating groove 751 and a second seating groove 752 are formed around the penetrating hole 753. The first and second seating grooves 751, 752 are areas for receiving a side surface projection 715 protruding from the latch 710 in the radially inward direction, and the side surface projection 715 of the latch 710 may be seated in the first seating groove 751 or the second seating groove 752 according to a movement of the lock main body 720, such that the movement of the lock main body 720 is temporarily fixed.

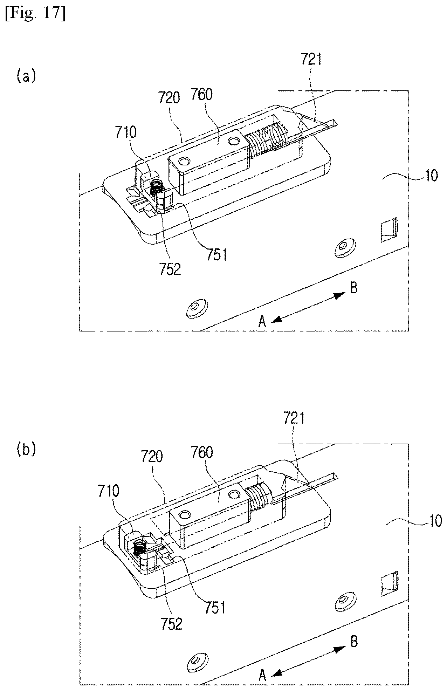

That is, as shown in FIG. 17(b), in a state where the lock main body 720 is retracted in the "B" direction, the latch 710 is locked into the first seating groove 751. When the lock main body 720 is pushed by the auxiliary push bar 630 and moves forward in the "A" direction, the latch 710 is locked into the second seating groove 752 as shown in FIG. 17(b). Since the latch 710 is locked into the second seating groove 752, the locking portion 720 is not retracted in the "B" direction and is temporarily fixed even when the push operation of the auxiliary push bar 630 is removed.

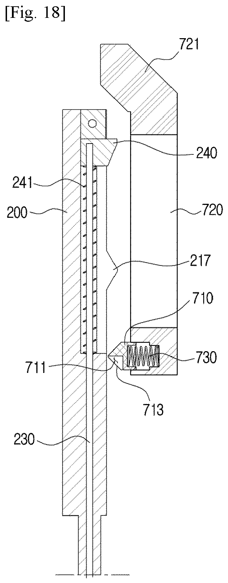

FIG. 18 is a view illustrating operations of locking and unlocking of the needle channel and the core shaft locking portion according to an embodiment. When a force is not exerted to the latch 710, the latch 710 protrudes from the lock main body 720 (in the radially inward direction of the needle channel bundle 1000) and comes into contact with the lever 240 when the lever 240 slides up and down. In this case, a groove 711 of the latch 710 locks the lever 240 and restrains the lever 240 from moving up. That is, when the lever 240 descends from top to bottom of the latch 710, the lever 240 may descend while pushing the latch 710 toward the lock main body 720, but, when the lever 240 ascends from bottom to top of the latch 710, an upper surface of the lever 240 is locked into the groove 710 of the latch 710 and does not ascend anymore.

As shown in FIG. 10, the body portion 210 of the needle channel 200 includes projections 217 protruding in the radially outward direction adjacent to both sides of the slot 215. A horizontal width of the latch 710 of the core shaft locking portion 700 is set to interfere with not only a movement of the lever 240 but also a vertical movement of the projection 217. That is, as shown in FIGS. 16, 17(b), 17(b), and 18, a contact surface of the latch 710 coming into contact with the projection 217 has an inclined surface 713. Accordingly, when the needle channel 200 ascends from bottom to top of the latch 710 or descends from top to bottom, the projection 217 ascends/descends while pushing the latch 710 toward the lock main body 720.

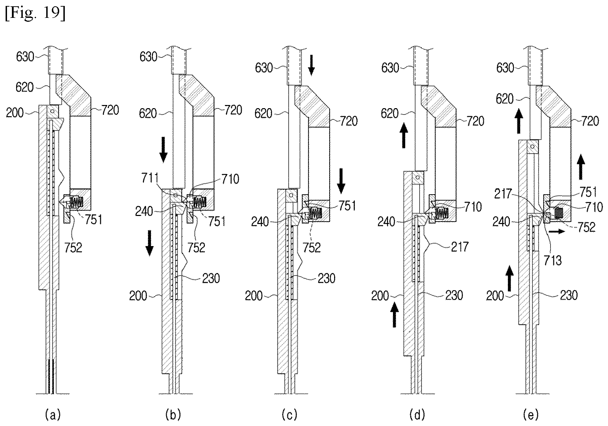

Hereinbelow, the operation of moving forward and retracting the needle channel according to an embodiment will be described with reference to FIGS. 19(a), 19(b), 19(c), 19(d), 19(e).

FIG. 19(a) illustrates a state before the needle channel 200 attached to the needle channel bundle 1000 moves down. In this state, both the lever 240 and the projection 217 of the needle channel 200 are positioned above the latch 710 of the core shaft locking portion 700, and as shown in FIG. 15(a), since the step portion 542 of the sliding block 540 catches the push block 610 and pulls back, the main spring 600 and the push block 610 coupled thereto, and the main push bar 620 are temporarily stopped in the retracted state.

In this case, when the sliding block 540 is slightly retracted, the locking of the sliding block 540 by the step portion 542 is removed and the main push bar 620 pushes down the needle channel 200 by the elastic force of the spring 600 as shown in FIG. 15(b). Then, the needle channel 200 moves down (FIG. 19(b)). When the needle channel 200 moves down, the projection 217 and the lever 240 come into contact with the inclined surface of the latch 710, and thus the needle channel 200 and the lever 240 may move down while pushing the latch 710 toward the latch receiving space 725. As the needle channel 200 moves down by the main spring 600 and moves forward as described above, the needle 220 penetrates through the scalp and is inserted by the first predetermined distance d1 as shown in FIG. 3(a). In the state where the needle channel 200 moves down, the upper surface of the lever 240 is locked into the groove 711 of the latch 710 and does not move up and is fixed.

Thereafter, as shown in FIG. 19(c), the sliding block 540 pushes the auxiliary push bar 630 and the auxiliary push bar 630 moves down. Accordingly, the auxiliary push bar 630 pushes down the lock main body 720 of the core shaft locking portion 700, and accordingly, the core shaft 230 further moves forward by the second distance as shown in FIG. 3(b), and the hair follicle 5 in the needle 220 penetrates through the scalp and is implanted therein. In the state where the lock main body 720 moves forward by the second distance, the lock main body 720 is seated in the second seating groove 752 and is fixed.

Next, referring to FIG. 19(d), the sliding block 540 moves up by the second driving motor 510. When the sliding block 540 moves up and is retracted, the locking projection 650b of the locking portion 650 of the push block 610 is locked into the step portion 542 of the sliding block, and accordingly, the push block 610 and the main push bar 620 are also retracted along with the sliding block 540, and the main spring 600 is compressed, becoming shorter.

At the same time, the force exerted by the main push bar 620 to press the needle channel 200 forward is removed, and thus, the needle channel 200 is pushed by the elevation portion 150 and moves up. As a result, the main push bar 620 and the needle channel 200 move up altogether as shown in FIG. 19(d).

As the needle channel 200 moves up, the needle 200 coupled to the lower end of the needle channel 200 moves up and is retracted. That is, as shown in FIG. 3(c), the retracting operation of the needle 220 starts. However, in the initial state of the retracting operation, the core shaft 230 is locked by the latch 710 and the latch 710 is locked into the second seating groove 752 as shown in FIG. 19(c), and accordingly, the core shaft 230 does not move up.

Thereafter, when the needle channel 200 continues moving up and moves up by the third predetermined distance, the projection 217 of the needle channel 200 comes into contact with the latch 710 and pushes the latch 710 toward the lock main body 720 as shown in FIG. 19(e). Accordingly, as the latch 710 is retracted toward the lock main body 720, the locking of the lever 240 by the latch 710 is removed, and simultaneously or in sequence, the locking of the latch 710 into the second seating groove 752 is removed, and accordingly, the lever 240 and the core shaft 230 move up due to the elastic force of the spring 241, and accordingly, the needle 220 and the core shaft 230 are retracted all together as shown in FIG. 3(d). In this case, the lock main body 720 moves up and returns to its original position due to the elastic force of the spring 704 (830).

As described above, the needle channel 200 moves up and the core shaft 230 also moves up, and returns to its original position as shown in FIG. 19(a), and thereafter, when the needle channel bundle 1000 is rotated by the first driving motor 300 as much as one needle channel, the operations of FIGS. 19(a), 19(b), 19(c), 19(d), 19(e) are repeated.

## Claims

1. A hair transplanter comprising:
a needle channel bundle (1000) comprising a center shaft (100), and a plurality of needle channels (200) coupled to an outer circumference of the center shaft (100) to be slidable up and down;
a first spring (600) configured to push down and move forward one of the plurality of needle channels (200); and
wherein each of the needle channels (200) comprises a body portion (210) having a tubular inner space, a needle (220) coupled to a lower portion of the body portion, and a core shaft (230) disposed in the tubular inner space to be slidable,
**characterized in that**
a second spring (190) configured to retract the needle channel (200) in an upward direction,
the hair transplanter further comprising a core shaft locking portion (700) which temporarily fixes a position of the core shaft (230) of the needle channel (200) which moves forward by the first spring,
wherein, in the state where the core shaft locking portion (700) temporarily fixes the position of the core shaft (230) of the needle channel (200) which moves forward, the hair transplanter is configured to push down the core shaft locking portion (700) by a predetermined distance and to further move forward the core shaft (230) by a predetermined distance.

2. The hair transplanter of claim 1, further comprising:
a push block (610) attached to a front end of the first spring (600); and
a first push bar (620) disposed on and attached to a front end of the push block (610) in a longitudinal direction,
wherein the first push bar (620) is configured to move forward the needle channel (200) by elasticity of the first spring (600).

3. The hair transplanter of claim 2, further comprising:
a driving motor (510) disposed in the hair transplanter;
a lead screw (530) coupled to a driving shaft of the driving motor (510) and having a screw thread formed on an outer circumference thereof; and
a sliding block (540) coupled to the lead screw (530) to move forward or backward by rotation of the lead screw (530),
wherein the push block (610) is configured to be retracted by the sliding block (540).

4. The hair transplanter of claim 3, wherein a step portion (542) is formed on a surface of the sliding block (540),
wherein the push block (610) further comprises a locking portion (650) moving in a radial direction, and
wherein, when the sliding block (540) is retracted, the step portion (542) is configured to lock the locking portion (650) and to make the sliding block (540) and the push block (610) be retracted all together.

5. The hair transplanter of claim 1, further comprising an elevation portion (150) which is positioned in a radially inward direction of the hair transplanter with respect to the needle channel (200), and moves in a longitudinal direction of the hair transplanter, and
wherein the elevation portion (150) is configured to push the needle channel (200) and to retract the needle channel (200) by the second spring (170).

6. The hair transplanter of any one of the previous claims, wherein, when the needle channel (200), which moves forward, is retracted, the core shaft locking portion (700) fixes the core shaft until the needle channel (200) is retracted by a predetermined distance, and the core shaft locking portion (700) unlocks the core shaft (230) after the needle channel (200) is retracted by the predetermined distance.

## Patentansprüche

1. Haartransplantations-Vorrichtung, die umfasst:
ein Nadelkanal-Bündel (1000), das einen Mittelschaft (100) sowie eine Vielzahl von Nadelkanälen (200) umfasst, die mit einem Außenumfang des Mittelschafts (100) so verbunden sind, dass sie nach oben und unten verschoben werden können;
eine erste Feder (600), die so ausgeführt ist, dass sie einen der Vielzahl von Nadelkanälen (200) nach unten drückt und vorwärts bewegt; und
wobei jeder der Nadelkanäle (200) einen Körperabschnitt (210) mit einem röhrenförmigen Innenraum, eine Nadel (220), die mit einem unteren Abschnitt des Körperabschnitts verbunden ist, sowie einen Kernschaft (230) umfasst, der in dem röhrenförmigen Innenraum so angeordnet ist, dass er verschoben werden kann,
**dadurch gekennzeichnet, dass**
eine zweite Feder (190) so ausgeführt ist, dass sie den Nadelkanal (200) in einer Aufwärtsrichtung einzieht,
wobei die Haartransplantations-Vorrichtung des Weiteren einen Kernschaft-Arretierabschnitt (700) umfasst, der eine Position des Kernschafts (230) des Nadelkanals (200), der sich aufgrund der ersten Feder vorwärts bewegt, temporär fixiert,
die Haartransplantations-Vorrichtung so ausgeführt ist, dass sie in dem Zustand, in dem der Kernschaft-Arretierabschnitt (700) die Position des Kernschafts (230) des Nadelkanals (200), der sich vorwärts bewegt, temporär fixiert, den Kernschaft-Arretierabschnitt (700) um eine vorgegebene Distanz nach unten drückt und den Kernschaft (230) um eine vorgegebene Distanz weiter vorwärts bewegt.

2. Haartransplantations-Vorrichtung nach Anspruch 1, die des Weiteren umfasst:
einen Drückblock (610), der an einem vorderen Ende der ersten Feder (600) angebracht ist; sowie
eine erste Drückstange (620), die an einem vorderen Ende des Drückblocks (610) in einer Längsrichtung angeordnet und angebracht ist,
wobei die erste Drückstange (620) so ausgeführt ist, dass sie den Nadelkanal (200) durch Elastizität der ersten Feder (600) vorwärts bewegt.

3. Haartransplantations-Vorrichtung nach Anspruch 2, die des Weiteren umfasst:
einen Antriebsmotor (510), der in der Haartransplantations-Vorrichtung angeordnet ist;
eine Gewindespindel (530), die mit einer Antriebswelle des Antriebsmotors (510) gekoppelt ist und ein an ihrem Außenumfang ausgebildetes Schraubengewinde aufweist; sowie
einen Gleitblock (540), der mit der Gewindespindel (530) gekoppelt ist und sich durch Drehung der Gewindespindel (530) vorwärts oder rückwärts bewegt,
wobei der Drückblock (610) so ausgeführt ist, dass er durch den Gleitblock (540) eingezogen wird.

4. Haartransplantations-Vorrichtung nach Anspruch 3, wobei ein Absatzabschnitt (542) an einer Oberfläche des Gleitblocks (540) ausgebildet ist,
der Drückblock (610) des Weiteren einen Arretierabschnitt (650) umfasst, der sich in einer radialen Richtung bewegt, und
der Absatzabschnitt (542) so ausgeführt ist, dass er, wenn der Gleitblock (540) eingezogen wird, den Arretierabschnitt (650) arretiert und bewirkt, dass der Gleitblock (540) und der Drückblock (610) zusammen eingezogen werden.

5. Haartransplantations-Vorrichtung nach Anspruch 1, die des Weiteren einen Erhöhungsabschnitt (150) umfasst, der in einer radial nach innen verlaufenden Richtung der Haartransplantations-Vorrichtung in Bezug auf den Nadelkanal (200) positioniert ist und sich in einer Längsrichtung der Haartransplantations-Vorrichtung bewegt, und
wobei der Erhöhungsabschnitt (150) so ausgeführt ist, dass er den Nadelkanal (200) drückt und den Nadelkanal (200) mittels der zweiten Feder (170) einzieht.

6. Haartransplantations-Vorrichtung nach einem der vorangehenden Ansprüche, wobei, wenn der Nadelkanal (200), der sich vorwärts bewegt, eingezogen wird, der Kernschaft-Arretierabschnitt (700) den Kernschaft fixiert, bis der Nadelkanal (200) um eine vorgegebene Distanz eingezogen ist, und der Kernschaft-Arretierabschnitt (700) den Kernschaft (230) entarretiert, nachdem der Nadelkanal (200) um die vorgegebene Distanz eingezogen ist.

## Revendications

1. Dispositif de transplantation capillaire comprenant :
un faisceau de canaux d'aiguille (1000) comprenant une tige centrale (100), et une pluralité de canaux d'aiguille (200) accouplés à une circonférence externe de la tige centrale (100) pour pouvoir coulisser vers le haut et vers le bas ;
un premier ressort (600) conçu pour pousser vers le bas et déplacer vers l'avant l'un de la pluralité des canaux d'aiguille (200) ; et
chacun des canaux d'aiguille (200) comprenant une portion formant corps (210) ayant un espace interne tubulaire, une aiguille (220) accouplée à une portion inférieure de la portion formant corps, et une tige centrale (230) disposée dans l'espace interne tubulaire pour pouvoir coulisser,
**caractérisé en ce que**
un second ressort (190) conçu pour rétracter le canal d'aiguille (200) dans un sens vers le haut,
le dispositif de transplantation capillaire comprenant en outre une portion de verrouillage de tige centrale (700) qui fixe temporairement une position de la tige centrale (230) du canal d'aiguille (200) qui se déplace vers l'avant par le premier ressort,
dans l'état où la portion de verrouillage de tige centrale (700) fixe temporairement la position de la tige centrale (230) du canal d'aiguille (200) qui se déplace vers l'avant, le dispositif de transplantation capillaire est conçu pour pousser vers le bas la portion de verrouillage de tige centrale (700) d'une distance prédéterminée et pour déplacer en outre vers l'avant la tige centrale (230) d'une distance prédéterminée.

2. Dispositif de transplantation capillaire selon la revendication 1, comprenant en outre :
un bloc de poussée (610) fixé à une extrémité avant du premier ressort (600) ; et
une première barre de poussée (620) disposée sur, et fixée à, une extrémité avant du bloc de poussée (610) dans un sens longitudinal,
la première barre de poussée (620) étant conçue pour déplacer vers l'avant le canal d'aiguille (200) par une élasticité du premier ressort (600).

3. Dispositif de transplantation capillaire selon la revendication 2, comprenant en outre :
un moteur d'entraînement (510) disposé dans le dispositif de transplantation capillaire ;
une vis-mère (530) accouplée à un arbre d'entraînement du moteur d'entraînement (510) et ayant un filetage de vis formé sur sa circonférence externe ; et
un bloc coulissant (540) accouplé à la vis-mère (530) pour se déplacer vers l'avant ou vers l'arrière par rotation de la vis-mère (530),
le bloc de poussée (610) étant conçu pour être rétracté par le bloc coulissant (540).

4. Dispositif de transplantation capillaire selon la revendication 3, une portion formant marche (542) étant formée sur une surface du bloc coulissant (540),
le bloc de poussée (610) comprenant en outre une portion de verrouillage (650) se déplaçant dans un sens radial, et
lorsque le bloc coulissant (540) est rétracté, la portion formant marche (542) est conçue pour verrouiller la portion de verrouillage (650) et pour faire se rétracter tous ensemble le bloc coulissant (540) et le bloc de poussée (610).

5. Dispositif de transplantation capillaire selon la revendication 1, comprenant en outre une portion d'élévation (150) qui est positionnée dans un sens radialement vers l'intérieur du dispositif de transplantation capillaire par rapport au canal d'aiguille (200), et qui se déplace dans un sens longitudinal du dispositif de transplantation capillaire, et
la portion d'élévation (150) étant conçue pour pousser le canal d'aiguille (200) et pour rétracter le canal d'aiguille (200) par le second ressort (170).

6. Dispositif de transplantation capillaire selon l'une quelconque des revendications précédentes, lorsque le canal d'aiguille (200), qui se déplace vers l'avant, est rétracté, la portion de verrouillage de tige centrale (700) fixe la tige centrale jusqu'à ce que le canal d'aiguille (200) est rétracté d'une distance prédéterminée, et la portion de verrouillage de tige centrale (700) déverrouille la tige centrale (230) après que le canal d'aiguille (200) est rétracté d'une distance prédéterminée.
